# EUROPEAN PATENT APPLICATION

(11) **EP 0 774 259 A1**
(43) Date of publication of application: **21.05.1997**
(21) Application number: 96120163.9
(22) Date of filing: 05.12.1991
(51) Int. Cl.: A61K 31/70, C07H 19/167

(54) **New use of organic compounds**

(30) Priority: 07.12.1990 DE 4039060; 26.07.1991 GB 9116212
(62) Divisional of application: 91810942.2
(71) Applicant: Novartis AG, 4058 Basel (CH); SANDOZ-PATENT-GMBH, 79539 Lörrach (DE); SANDOZ-ERFINDUNGEN Verwaltungsgesellschaft m.b.H., 1235 Wien (AT)
(72) Inventor: Gadient, Fulvio, 4127 Birsfelden (CH); Milavec, Maria, 68128 Village-Neuf (FR); Müller, Else, 4436 Oberdorf (CH)

(57) **Abstract**

Use of 2'-0-alkyl-adenosine-derivatives disclosed in the British Patent No. 2226027 and the European Patent application 378518 of formula 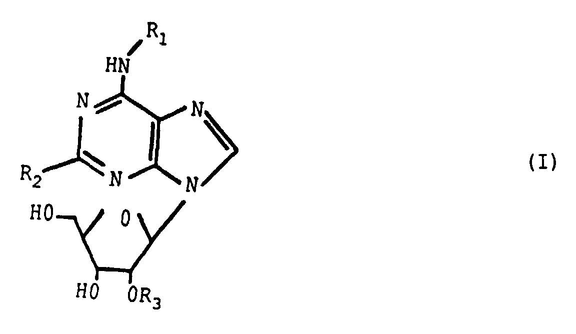 wherein R₁, R₂ and R₃ have the significances given in claim 1 for the manufacture of a medicament for the treatment of neurodegenerative disorders, certain peripheral neuropathies such as diabetic neuropathy and of disorders associated with peripheral vascular disease and/or disorders associated with neuronal degeneration, hypertriglyceridemia, elevated free fatty acids and/or type I and type II diabetes, arrhythmias and for protection against myocardial infarction.
Certain compounds of formula (I) can be used for lowering high blood lipid level.

## Description

This is a divisional application of European patent application no. 91810942.2.

The present invention relates to new use of 2'-O-alkyl-adenosine-derivatives disclosed in the British Patent Application No. 2226027 A and the European Patent Applications No. 378518 and 269574 and to pharmaceutical compositions containing such compounds and to their use in medicine.

From the above British Patent it is known that the compounds can be used as antihypertensive agents and as coronary vasodilators.

It is mentioned that both the inhibition of renin secretion and of release of noradrenaline from nerve endings and the direct vasodilation contribute to anti-hypertensive activity.

Furthermore it is disclosed that the compounds protect the vascular endothelium by inhibiting both thrombocyte aggregation and activation of leucocytes. They also lower the blood lipid levels.

Further some of the above 2'-O-alkyladenosine-derivatives also have a protective effect against diseases caused by hypertension such as congestive heart failure, myocardial infarction or sudden cardiac death and renal insufficiency (see Europ. Pat. Appl. No. 378518 and 269574).

It has now been found that the compounds of the above British Patent Appl. No. 2226027 and of the Europ. Pat. Appln. No. 378518 and 269574 are surprisingly of potential use for the treatment of peripheral vascular diseases such as intermittent claudication and Raynaud's disease.

The compounds are also active in the treatment of arrhythmias. As adenosine A1 receptor agonists they restore sinus rhythm in supraventricular tachycardias and return ventricular tachycardias induced by β-adrenergic stimulation to normal rhythm.

As adenosine A1 receptor agonists the compounds mimic the effect of "preconditioning" the procedure whereby a brief period of ischaemia renders the heart resistant to infarction from subsequent ischaemia. They can, therefore, be used to protect the heart against infarction.

The compounds are also indicated to have neuroprotectant activity. They are therefore useful in the treatment of neurodegenerative disorders, certain peripheral neuropathies such as diabetic neuropathy and the prophylaxis of peripheral vascular disorders associated with neuronal degeneration.

The compounds lower plasma insulin without influencing glucose tolerance. They potentiate the insulin effect on glucose uptake in adipose tissue. The compounds lower plasma free fatty acids. The insulin sparing effect and/or action to lower free fatty acids makes them useful for the treatment of type I and type II diabetes.

The compounds lower plasma triglycerides and free fatty acids and hence are useful in conditions where triglycerides and free fatty acids are increased.

Finally, the compounds show good metabolic stability.

Accordingly, the invention provides a method for the treatment of neurodegenerative disorders, certain peripheral neuropathies such as diabetic neuropathy and of disorders associated with peripheral vascular disease and/or disorders associated with neuronal degeneration, hypertriglyceridemia, elevated free fatty acids and/or type I and type II diabetes, arrhythmias and for protection against myocardial infarction, which method comprises the administration of an effective, non-toxic amount of a compound of formula: 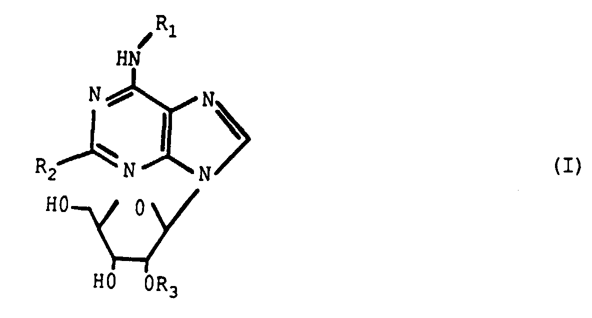 wherein
- R₁: signifies hydrogen, (C₁₋₄)alkyl, allyl; methallyl; a straight-chain or branched (C₃₋₇)alkinyl, (C₃₋₈)cycloalkyl, phenyl being independently of one another mono- or disubstituted by halogen with an atomic number of 9 to 35, (C₁₋₄)alkyl, (C₁₋₄)alkoxy or CF₃; or phenyl (C₁₋₄)alkyl whereby the phenyl ring is optionally independently of one another mono- or disubstituted by halogen with an atomic number of 9 to 35, (C₁₋₄)alkyl, (C₁₋₄)alkoxy or CF₃; (C₁₋₄)alkyl having at least one hydroxy group or at least two phenyl groups, a bicycloalkyl group, a naphtyl(C₁₋₄₎alkyl-group, an acenaphthylenyl(C₁₋₄)alkyl-group or a group of formula 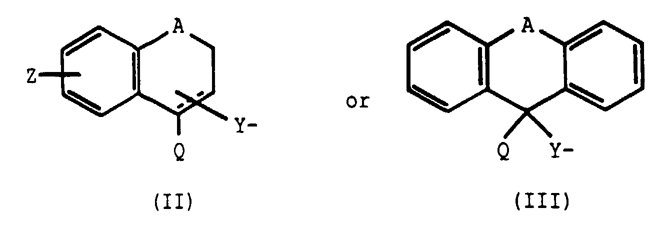 wherein
Z = hydrogen, a hydroxy group or a (C₁₋₄)alkoxy group.
Q = hydrogen or hydroxy,
A = -CH₂-, -0-, -S- or a direct bond,
Y = -(CH₂)ₙ- or a direct bond
n = 1-3
and the broken line in (II) represents an optional bond.
- R₂: is hydrogen, (C₁₋₄)alkyl, amino, (C₃₋₅)cycloalkyl or halogen with an atomic number of 9 to 35 and
- R₃: is (C₁₋₄)alkyl
to a person in need of such treatment.

The compounds of formula (I) can be used as such, as hydrates or addition products with other solvents like ethanol, certain compounds of formula (I) can be used in form of salts.

In an other aspect, the invention provides the use of a compound of formula (I) or a hydrate or an addition product with other solvents or salts thereof, for the manufacture of a medicament for the treatment of neurodegenerative disorders, certain peripheral neuropathies such as diabetic neuropathy and of disorders associated with peripheral vascular disease and/or disorders associated with neuronal degeneration, hypertriglyceridemia, elevated free fatty acids and/or type I or type II diabetes, arrhythmias and for protection against myocardial infarction.

The present invention also provides a pharmaceutical composition comprising a compound of formula I or a hydrate or an addition product with other solvents or salts thereof and a pharmaceutically acceptable carrier therefore.

The invention provides especially the use of a compound of formula (I) wherein R₁ represents (C₃₋₈)cycloalkyl especially cyclohexyl, R₂ hydrogen and R₃ (C₁₋₄)alkyl especially methyl or a hydrate thereof for the manufacture of a medicament for the treatment of neurodegenerative disorders, certain peripheral neuropathies such as diabetic neuropathy and of disorders associated with peripheral vascular disease and/or disorders associated with neuronal degeneration, hypertriglyceridemia, elevated free fatty acids and/or type I and type II diabetes, arrhythmias and for protection against myocardial infarction.

### Compounds of formula (Ia)

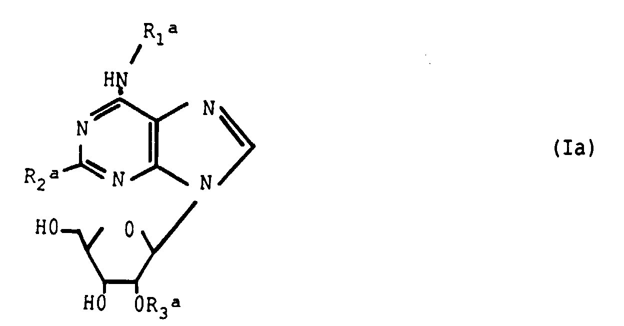

wherein
- R₁^{a}: is hydrogen, (C₁₋₄)alkyl having optionally at least one hydroxy group or at least two phenyl groups, bicycloalkyl naphthyl(C₁₋₄)alkyl, acenapthylenyl-(C₁₋₄)alkyl or a group of the above formula 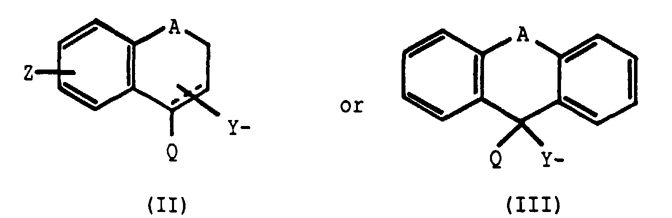 wherein
Z, Q, A, Y and n are defined above
- R₂^{a}: is hydrogen, amino, (C₁₋₄)alkyl or halogen with an atomic number of 9 to 35
- R₃^{a}: is a (C₁₋₄)alkyl-group
are also very active in lowering blood lipid levels.

Accordingly compounds of formula (Ia) are useful for treatment of disorders associated with high blood lipid levels which treatment comprises the administration of an effective, non-toxic amount of a compound of formula (Ia) to a person in need of such treatment.

Compounds of formula (Ia) can be used for the manufacture of medicaments for the treatment of high blood lipid level.

The following compounds 1 to 13 of formula (I), wherein R₁, R₂ and R₃ are as below being disclosed in UK patent application 2226027 A. The following compounds 14 to 41 of formula (I) wherein R₁, R₂ and R₃ are as below being disclosed in Europ. Patent application No. 378 518 and the following compounds 42 to 54 of formula (I) wherein R₁, R₂ and R₃ are as below being disclosed in Europ. Patent Application No. 269 574. They are especially advantageous in the use according to the invention.

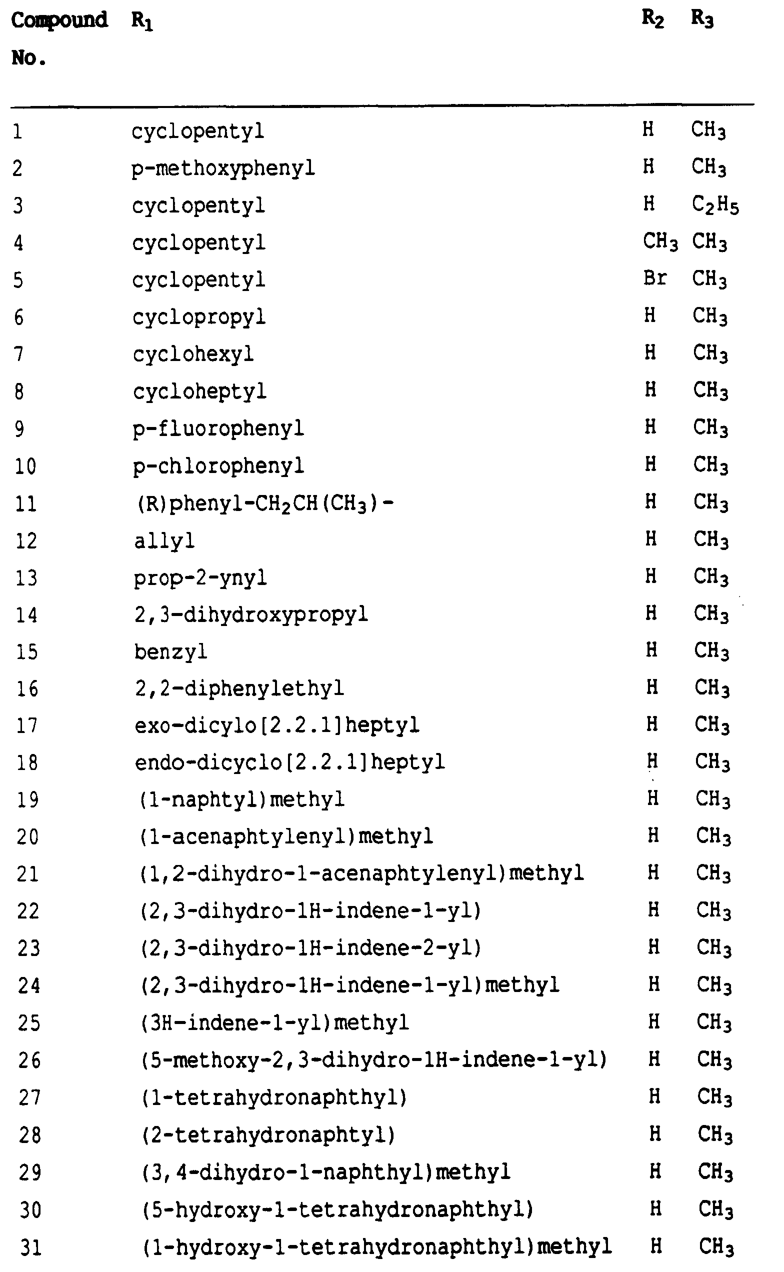

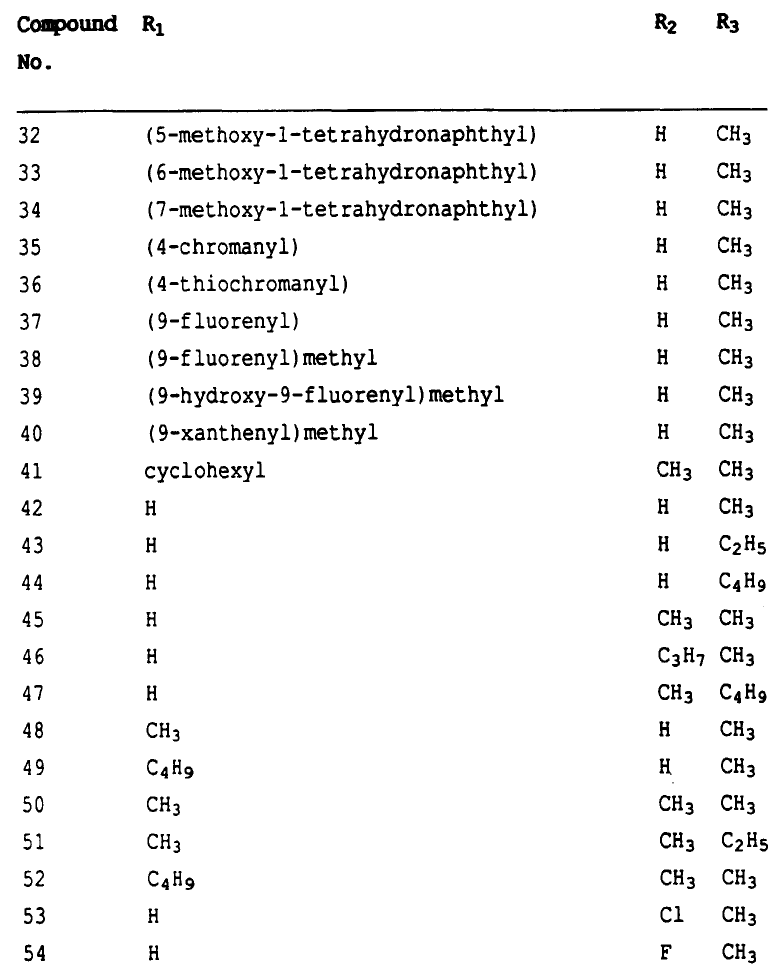

The most important compound for the use according to the instant invention is the compound No. 7 being preferably used in form of its hydrate. This hydrate form of compound No. 7 is new and forms a part of the instant invention. The hydrate is formed by treatment of the known monoetherat of compound No. 7 with an aqueous solvent.

Especially preferred is the 1.5 hydrate form.

The 1.5 hydrate of the compound No. 7 named 6-cyclohexyl-2'-0-methyl-adenosine 1.5 hydrate can be prepared in the following manner: 0,7 g 6-chloro-9-purinyl-2'-0-methyl-D-ribose are heated in 30 ml of cyclohexylamine during 2 hours to 80°. The mixture is subsequently evaporated to dryness under reduced pressure. The residue obtained is chromatographed on silica gel using a mixture of methylenechloride/ethanol 95:5 as eluent. The purified product is crystallized form methylenechloride/diethylether. As the product contains diethylether that can not be removed even in high vacuum it is crystallized from ethanol/water. The thus obtained 6-cyclohexyl-2'-0-methyl-adenosine 1.5 hydrate has a melting point of 88-91°, [α]_{D}²⁰ = -57,2 ° (c = 1 in dimethylformamide).

| **Elementary Analysis** | | |
|---|---|---|
| **Calculated :** | C | 52,3% |
| | H | 7,2% |
| | N | 17,9% |
| | 0 | 22,5% |
| **Found :** | C | 52,5% |
| | H | 7,2% |
| | N | 18,0% |
| | 0 | 22,4% |

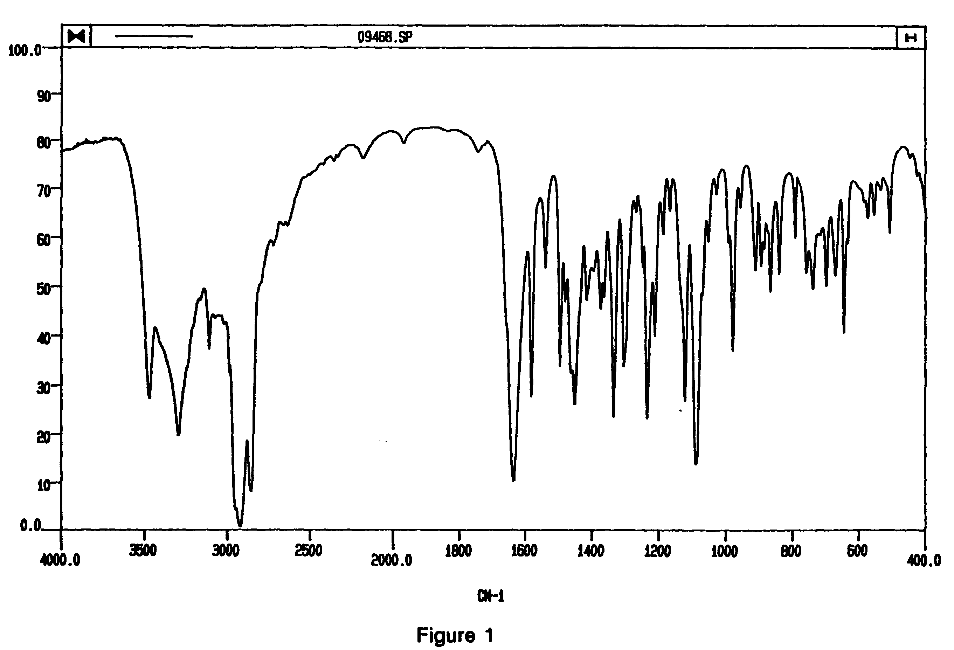

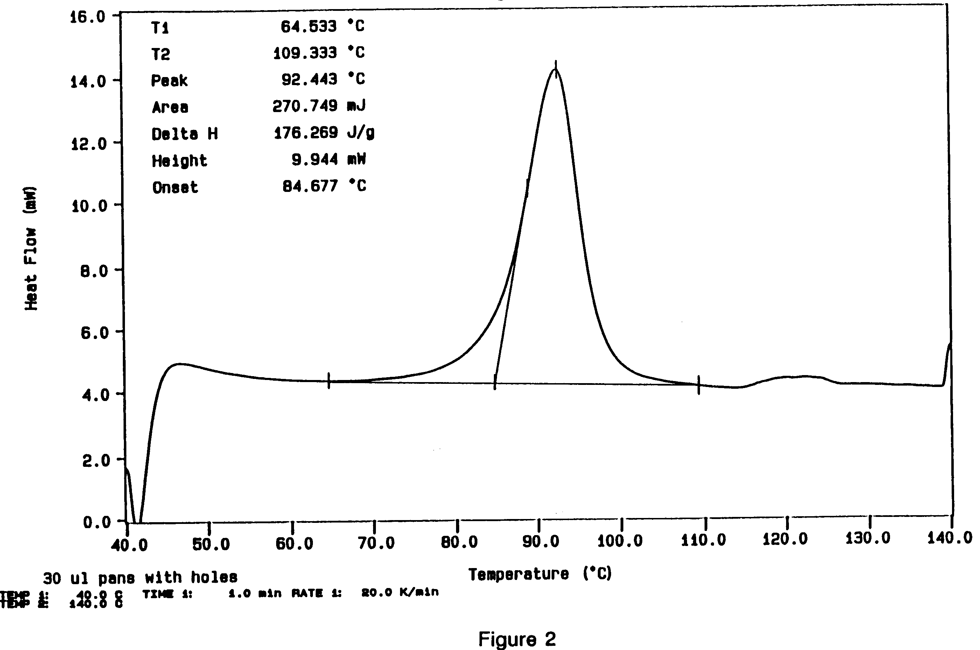

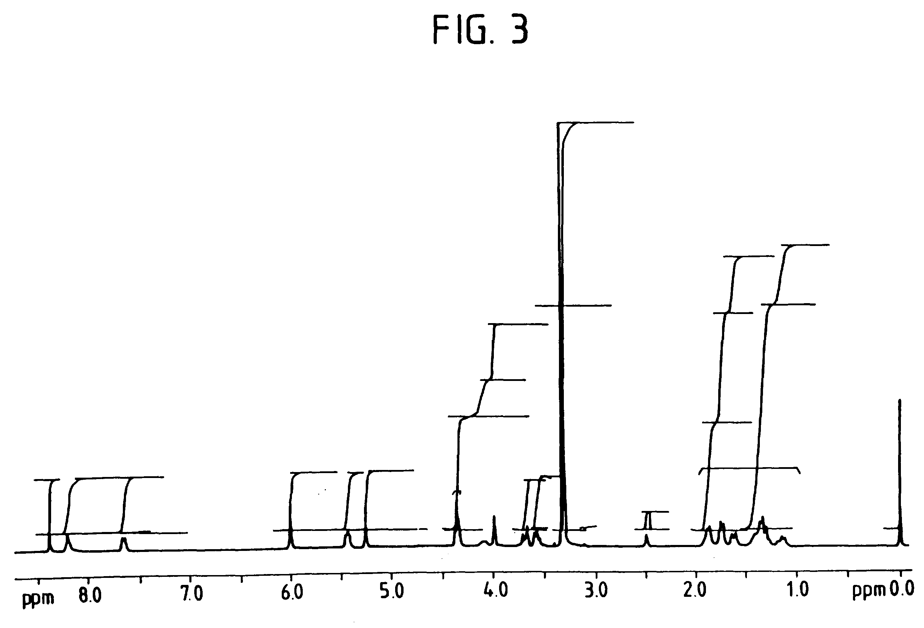

The IR spectrum of this 1.5 hydrate, the result of DSC thermal analysis and the results of NMR analysis are attached as figures 1, 2 and 3. The results of these measurements together with the results of elementary analysis show that according to the above process 6-cyclohexyl-2'-0-methyl-adenosine 1.5 hydrate has been prepared.

The compounds of formula (I) are preferably in pharmaceutically acceptable form or in the form of a hydrate or as addition products with other solvents like ethanol or as salts. By pharmaceutically acceptable form is meant, inter alia, of a pharmaceutically acceptable level of purity including normal pharmaceutical additives such as diluents and carriers, and excluding material considered toxic at normal dosage levels.

The salts can be salts with alkalimetals like sodium or potassium salts or acid addition salts like hydrochlorides.

The compounds of formula (I) may be formulated for administration by any suitable route, the preferred route depending upon the disorder for which treatment is required, and preferably in unit dosage form or in a form that a human patient may administer to himself in a single dosage. Advantageously, the composition is suitable for oral, rectal, topical, parenteral, intravenous or intramuscular administration.

Preparations may be designed to give slow release of the active ingredient.

The compositions of the invention may be in the form of tablets, capsules, sachets, vials, powders, granules, lozenges, suppositories, reconstitutable powders, or liquid preparations such as oral or sterile parenteral solutions or suspensions. Topical formulations are also envisaged where appropriate.

In order to obtain consistency of administration it is preferred that a composition of the invention is in the form of a unit dose.

Unit dose presentation forms for oral administration may be tablets and capsules and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol, or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulphate.

The solid oral compositions may be prepared by conventional methods of blending, filling, tabletting or the like. Repeated blending operations may be used to distribute the active agent throughout those compositions employing large quantities of fillers.

Such operations are of course conventional in the art. The tablets may be coated according to methods well known in normal pharmaceutical practice, in particular with an enteric coating.

Oral liquid preparations may be in the form of, for example, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; non-aqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents.

For parenteral administration, fluid unit dosage forms are prepared utilizing the compound and a sterile vehicle, and, depending on the concentration used, can be either suspended or dissolved in the vehicle. In preparing solutions the compound can be dissolved in polyethyleneglycol or ethanol and diluted with water for injection and filter sterilized before filling into a suitable vial or ampoule and sealing. Advantageously, adjuvants such as a local anaesthetic, a preservative and buffering agents can be dissolved in the vehicle. Parenteral suspensions are prepared in substantially the same manner, except that the compound is suspended in the vehicle instead of being dissolved, and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

The compositions may contain from 0.1% to 99% by weight, preferably from 10-60% by weight of the active material, depending on the method of administration.

Compounds of formula (I), or a hydrate or an addition product with other solvents or salts thereof, may also be administered as a topical formulation in combination with conventional topical excipients.

Topical formulations may be presented as, for instance, ointments, creams or lotions, impregnated dressings, gels, gel sticks, spray and aerosols, and may contain appropriate conventional additives such as preservatives, solvents to assist drug penetration and emollients in ointments and creams. The formulations may contain compatible conventional carriers, such as cream of ointment bases and ethanol or oleyl alcohol for lotions.

Suitable cream, lotion, gel, stick, ointment, spray or aerosol formulations that may be used for compounds of formula (I) or a hydrate or an addition product with other solvents or salts thereof, are conventional formulations well known in the art, for example, as described in standard text books of pharmaceutics and cosmetics, such as Harry's Cosmeticology published by Leonard Hill Books, Remington's Pharmaceutical Sciences, and the British and US Pharmacopoeias.

Suitably, the compound of formula (I), or a hydrate or an addition product with other solvents or salts thereof, will comprise from about 0.5 to 20% by weight of the formulation, favourably from about 1 to 10% for example 2 to 5%.

The dose of the compound used in the treatment of the invention will vary in the usual way with the seriousness of the disorders, the weight of the sufferer, and the relative efficacy of the compound. However, as a general guide suitable unit doses may be 0.1 to 1000 mg, such as 0.5 to 200, 0.5 to 100 or 0.5 to 10 mg, for example 0.5, 1, 2, 3, 4 or 5 mg; and such unit doses may be administered more than once a day, for example 2, 3, 4, 5 or 6 times a day, but preferably 1 or 2 times per day, so that the total daily dosage for a 70 kg adult is in the range of about 0.1 to 1000 mg, that is in the range of about 0.001 to 20 mg/kg/day, such as 0.007 to 3, 0.007 to 1.4, 0.007 to 0.14 or 0.01 to 0.5 mg/kg/day, for example 0.01, 0.02, 0.04, 0.05, 0.06, 0.08, 0.1 or 0.2 mg/kg/day; and such therapy may extend for a number of weeks or months.

When used herein the term "pharmaceutically acceptable" encompasses materials suitable for both human and veterinary use.

No toxicological effects have been established for the compounds of formula (I) in the above mentioned dosage ranges.

The following pharmacological tests have been effected to establish the activity of the compounds:
**a) Affinity for adenosine receptors**
Pig striatal membranes were prepared as previously described by H. Bruns et al. in Molecular Pharmacology 29 (1986) pages 331-344. ³H-NECA, a non-selective adenosine receptor agonist was used to label both A₁ and A₂ receptors. IC₅₀ values were derived from the displacement curves by weighted non-linear least-square curve fitting to the Langmuir equation and pK_{D} values calculated.
The results are presented in the following Table in agreement with the literature, CPA proved a potent and highly selective displacer of binding to the A₁ receptor, CV 1808 was a relatively weak but selective A₂ receptor ligand and CGS 21680 showed high potency and selectivity for the A₂ receptor. Compound No. 7 in the form of its 1.5 hydrate shows good affinity and high selectivity for the A₁ receptor.

**Table**

| Affinity of adenosine receptor ligands for A₁ and A₂ receptors | | | | |
|---|---|---|---|---|
| | **A**_{**1**} **(K**_{**D**}**;nM)** | **A**_{**2**} **(K**_{**D**}**;nM)** | **A**_{**1**}**:A**_{**2**} **selectivity** | **n** |
| CPA^{a} | 0.74 ± 0.01 | 33 ± 110 | 1260 | 6 |
| CV 1808^{a} | 2460 ± 757 | 269 ± 70 | 0.1 | 5 |
| CGS 21680^{a} | 4360 ± 1080 | 10 ± 4 | 0.004 | 4 |
| Compound No. 7 | 23 ± 2 | 24500 ± 5160 | 1090 | 5 |
| 1.5 H₂0 | | | | |
| CPA^{a} = Cyclopentyladenosine CV 1808^{a} = 2-Phenylaminoadenosine (Carbohydrates vol. 81 (1974) ref. 91898 K) CGS 21680^{a} = 2-[p-(2-Carboxyethyl)phenethylamino]-5'-N-ethyl carboxamido-adenosine (FASEB J, 1989, 3 (4) Ref. 4770 and 4773) | | | | |

**b) Arrhythmias**
Adenosine receptor activation reduces the incidence of supraventricular tachycardia and other arrhythmias. The test method is disclosed by C. Clarke et al. in The Pharmaceutical Journal Vol. 244, 595 to 597 (1990). In this test compound No. 7 is used in form of its 1.5 hydrate.
**c) Mean arterial blood pressure, bradycardia and peripheral vasodilation on anesthetized rats.**
The experiments were carried out on male Wistar rats, body weight 300-350 g (323 ± 3 g), under Inactin anaesthesia (150 mg/kg i.p.), according to the method of Schroeder et al. (Measurement of the cardiodynamics, haemodynamics and the ECG in the anesthetized rat, effects of catecholamines (dopamine and isoproterenol) in Budden et al. "The Rat Electrocardiogram in Pharmacology and Toxicology" Oxford Pergamon Press 1981, 155-9) Catheters were placed in the right jugular and right femoral veins, in the left ventricle (inserted via the right carotid artery), left femoral artery, and aorta (inserted through the right femoral artery). The following variables were measured or calculated: systolic, diastolic, and mean arterial blood pressure (mm Hg; left femoral artery, Statham pressure transducer P 23 Gb), pulse pressure (mm Hg), heart rate (beats/min; triggered from the blood pressure curve), rate of rise of left ventricular pressure (dP/dtₘₐₓ, mm Hg/s; Statham pressure transducer P 23 Gb), cardiac output (ml/min/100 g body weight, thermodilution method, right jugular vein and aorta), total peripheral resistance (dynes · s cm⁻⁵/100 g body weight), ECG lead III. Arterial blood pressure, left ventricular pressure, dP/dt ₘₐₓ, heart rate, and electrocardiogram were continuously recorded with a Schwarzer polygraph. The parameters were measured 30, 20, 10 and 2 min before administration of the substance and 1, 10, 30 and 60 min after injection of the drug into the right femoral vein. Compound No. 7 in form of its 1.5 hydrate was tested in doses of 0.003, 0.01 and 0.03 mg/kg whereby five animals per dose were used.
**d) Blood flow through ischaemic skeletal muscle**
Male normotensive rats (ca. 300 g) were anaesthetised with Evipan Na (160 mg/kg i.p.) and the right femoral artery was ligated. Three weeks after ligation the rats were anaesthetised with pentothal (Thiopental-Na, 40 mg/kg i.p.) and placed on a special holder for measurement of the resonances of phosphocreatine (PCr), inorganic phosphate (Pi), of the three phosphates of ATP, and of phosphomono-esters and sugar phosphates (PM) by use of a Bruker Biospec 47/15 spectrometer equipped with a a 4.7 T horizontal magnet of 15 cm clear bore. The blood supply to the right hindleg was then temporarily occluded for about 30-40 min by a tourniquet placed around the upper hindleg, until the PCr levels had fallen to about 1/3 of the original level. Thereafter the occlusion was released and the rate of recovery (τ₁) of PCr was followed. Thirty minutes later the same procedure was repeated without and after intravenous administration of the drug e.g. compound No. 7 in form of its 1.5 hydrate under investigation, the rate of recovery or the PCr level (τ₂) being followed again. The ratio of both recovery times (τ₂/τ₁) was taken as a parameter for the drug effect on the peripheral circulation. Some animals were tested with the sympathetic neurotoxin 6-hydroxydopamine, at the time of femoral artery ligation and the effects of compound No. 7 in the form of its 1.5 hydrate on the induced peripheral vascular insufficiency determined.
**e)** **Glucose Transport in Rat Adipocytes**
Adipocytes were isolated from the epididymal fat pads of normal chow-fed rats by digestion with collagenase. Cells (final concentration 2% v/v) were pre-incubated with adenosine deaminase (1 U/ml), test compounds e.g. compound No. 7 in form of its 1.5 hydrate and other additions as indicated for 30 min at 37°C. [3-³H]Glucose (final concentration, 50 µM, 0.5 µCi/ml) was then added, and incubation was continued for a further 60 min. Incorporation of radioactivity from [3-³H]-glucose into cell lipids (a measure of glucose transport) was evaluated by extraction of the cell suspension (0.5 ml) with 5 ml of toluene-based scintillant, followed by liquid scintillation counting; water-soluble metabolites and residual [3-³H] glucose remain in the aqueous phase and are not detected.
**f) Dy****slipidemias characterised by elevated serum triglycerides**
Several studies have shown a positive correlation between serum triglyceride levels and the risk for coronary heart disease (CHD) (Grundy, in Cholesterol and Atherosclerosis: Diagnosis and Treatment, Lippincott, Philadelphia (1990)). The value of reducing elevated triglyceride levels as an approach to reducing the risk of CHD emerged from the Helsinki Heart Study where, following treatment with gemfibrozil, the greatest reduction in serious coronary events occured in Type IIB hyperlipidemic patients in whom both LDL-cholesterol and total serum triglycerides were elevated. Compound No. 7 in the form of its 1.5 hydrate produced substantial and long-lasting falls in plasma free fatty acids and triglycerides in the Rhesus monkey.
**g)** **Protection against infarction by preconditioning**
Preconditioning (5 minutes of ischaemia followed by 10 minutes of recovery) renders the heart very resistant to infarction from subsequent ischaemia. The test method is disclosed in the article of G.S. Liu et al. in Circulation 84, 1, 350 to 356 (1991). In this test compound No. 7 is used in form of its 1.5 hydrate.

## Claims

1. Use of a compound of formula 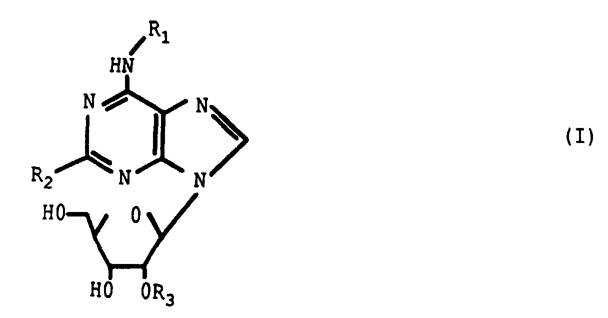 wherein
R₁ signifies hydrogen, (C₁₋₄)alkyl, allyl; methallyl; a straight-chain or branched (C₃₋₇)alkinyl, (C₃₋₈)cycloalkyl, phenyl being independently of one another mono- or disubstituted by halogen with an atomic number of 9 to 35, (C₁₋₄)alkyl, (C₁₋₄)alkoxy or CF₃; or phenyl (C₁₋₄)alkyl whereby the phenyl ring is optionally independently of one another mono- or disubstituted by halogen with an atomic number of 9 to 35, (C₁₋₄)alkyl, (C₁₋₄)alkoxy or CF₃; (C₁₋₄)alkyl having at least one hydroxy group or at least two phenyl groups, a bicycloalkyl group, a naphtyl(C₁₋₄)alkyl-group, an acenaphthylenyl(C₁₋₄)alkyl-group or a group of formula 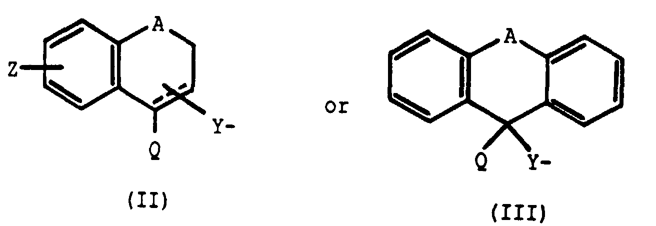
Z = hydrogen, a hydroxy group or a (C₁₋₄) alkoxy group.
Q = hydrogen or hydroxy,
A = -CH₂-, -0-, -S- or a direct bond,
Y = -(CH₂)ₙ- or a direct bond
n = 1-3
and the broken line in (II) represents an optional bond.
R₂ is hydrogen, (C₁₋₄)alkyl, amino, (C₃₋₅)cycloalkyl or halogen with an atomic number of 9 to 35 and
R₃ is (C₁₋₄)alkyl
or a hydrate or an addition product with other solvents or salts thereof for the manufacture of a medicament suitable for the treatment of neurogenerative disorders, certain peripheral neuropathies such as diabetic neuropathy and of disorders associated with peripheral vascular disease and/or disorders associated with neuronal degeneration, hypertriglyceridemia, elevated free fatty acids and/or type I and type II diabetes, arrhythmias and for protection against myocardial infarction.

2. Use according to claim 1 of a compound of formula (I) wherein R₁ is (C₃₋₈)cycloalkyl, R₂ is hydrogen and R₃ is (C₁₋₄)alkyl

3. Use according to claim 2 of a compound of formula (I) wherein R₁ is cyclohexyl, R₂ is hydrogen and R₃ is methyl.

4. Use of compound of formula 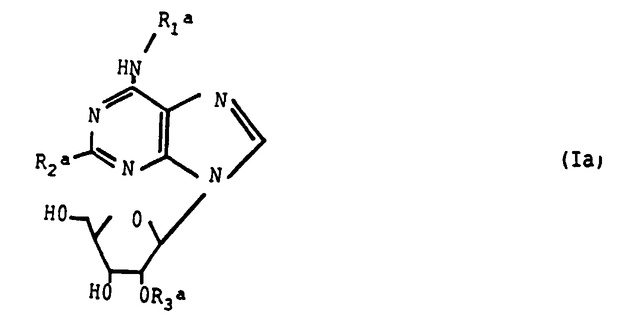 wherein
R₁^{a} is hydrogen, (C₁₋₄)alkyl having optionally at least one hydroxy group or at least two phenyl groups, bicycloalkyl, naphthyl(C₁₋₄)alkyl, acenapthylenyl(C₁₋₄)alkyl or a group of the formula 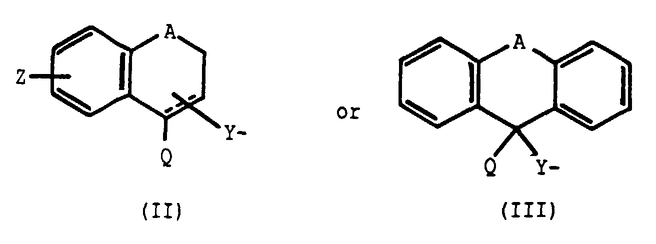 wherein
Z, Q, A, Y and n are defined in claim 1
R₂^{a} is hydrogen, amino (C₁₋₄)alkyl or halogen with an atomic number of 9 to 35 and
R₃^{a} is a (C₁₋₄)alkyl-group
or a hydrate or an addition product with other solvents or salts thereof for the manufacture of medicaments suitable for the treatment of high blood lipid level.

5. A pharmaceutical composition comprising a therapeutically effective amount of compounds of formula (I) according to claim 1 and a pharmaceutically acceptable carrier thereof suitable for treatment of neurodegenerative disorders, certain peripheral neuropathies such as diabetic neuropathy and of disorders associated with peripheral vascular disease and/or disorders associated with neuronal degeneration, hypertriglyceridemia, elevated free fatty acids and/or type I and type II diabetes, arrhythmias and for protection against myocardial infarction.

6. A pharmaceutical composition comprising a therapeutically effective amount of compounds of formula (Ia) according to claim 4 and a pharmaceutically acceptable carrier thereof suitable for the treatment of high blood lipid level.
